# EUROPÄISCHE PATENTANMELDUNG

(11) **EP 3 401 001 A1**
(43) Veröffentlichungstag der Anmeldung: **14.11.2018**
(21) Anmeldenummer: 17400026.5
(22) Anmeldetag: 12.05.2017
(51) Int. Cl.: B01D 53/14, C01B 3/52

(54) **VERFAHREN UND ANLAGE ZUR ABTRENNUNG VON BEGLEITGASEN AUS EINEM ROHSYNTHESEGAS**

(71) Anmelder: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Linicus, Matthias, 65817 Eppstein (DE); Jensen, Sandra, 60438 Frankfurt (DE)
(74) Vertreter: Dropsch, Holger

(57) **Zusammenfassung**

Verfahren und Anlage zur Abtrennung von Begleitgasen aus einem Rohsynthesegasstrom durch Waschen mit einem Absorptionsmittel, wobei das Absorptionsmittel in einem Kreisprozess gefahren wird, wobei zur Regenerierung das Absorptionsmittel auch durch eine ein Adsorbens enthaltende Festkörperschüttung geleitet wird.

## Beschreibung

### Gebiet der Erfindung

Die Erfindung betrifft ein Verfahren zur Abtrennung von Begleitgasen aus einem Rohsynthesegasstrom durch Waschen des Rohsynthesegasstroms mit einem flüssigen, physikalisch wirkenden Absorptionsmittel, wobei das Absorptionsmittel in einem Kreisprozess gefahren wird, in welchem es beim Waschen mit Begleitgasen beladen, anschließend durch Druckerniedrigung, Flashen und/oder Erwärmung desorbiert und dann zum Waschen des Rohsynthesegasstroms wiederverwendet wird.

Die Erfindung betrifft ebenso eine Anlage zum Betreiben des Verfahrens.

### Stand der Technik

Verfahren zur Abtrennung von unerwünschten Begleitstoffen aus einem Rohsynthesegasstrom, wie z. B. das Rectisol Verfahren, sind bekannt. Das Rectisol Verfahren wird in Ullmann's Encyclopedia of Industrial Chemistry, 6. Aufl. Bd. 15, S. 399 ff. grundsätzlich beschrieben. Dieses Verfahren dient dazu, aus Kohle oder Koks nach dem Festbettdruckvergasungsverfahren erzeugtes, hauptsächlich aus CO und H₂ bestehendes Rohsynthesegas durch Absorption der Begleitgase zu reinigen. Das Festbettdruckvergasungsverfahren wird in Ullmann's Encyclopedia of Industrial Chemistry, 6. Aufl. Bd. 15, S. 367ff grundsätzlich beschrieben. Das Rectisol Verfahren verwendet als Absorptionsmittel Methanol, wobei die Eigenschaft des Methanols ausgenutzt wird, dass seine Absorptionsfähigkeit für die Begleitstoffe mit abnehmender Temperatur stark zunimmt, während sie für CO und H₂ praktisch konstant bleibt. Bei den unerwünschten Begleitstoffen handelt es sich hauptsächlich um die Begleitgase Carbonylsulfid (COS), Schwefelwasserstoff (H₂S) und Kohlendioxid (CO₂).

Das als Absorptionsmittel verwendete Methanol wird bei diesem Verfahren über Regenerierungsanlagen im Kreis gefahren. In den Regenerierungsanlagen wird das beladene Methanol von den absorbierten Gasen auf physikalischem Wege befreit. Dabei wird in einem ersten Regenerationsschritt bevorzugt CO₂ durch Druckentspannung und/oder Strippen mit einem Gas, beispielsweise Stickstoff, aus dem beladenen Methanol-Absorptionsmittel entfernt. In einem zweiten Regenerationsschritt werden die schwefelhaltigen Gase, COS und H₂S, durch Erhitzen abgetrieben. Es wird angestrebt, ein möglichst CO₂-armes COS/H₂S-Gas zu erzeugen, da dessen wirtschaftlich interessante Weiterverarbeitung durch eine Vermischung mit CO₂ beeinträchtigt wird.

Beim Rectisol Verfahren wird zwischen dem Standard- und dem selektiven Rectisol-Verfahren unterschieden.

Beim sogenannten Standard-Rectisol-Verfahren werden die Begleitgase COS/H₂S und das CO₂ gemeinsam, in einem Absorptionsschritt aus dem Rohsynthesegas abgetrennt. Beim sogenannten selektiven Rectisol-Verfahren werden die schwefelhaltigen Begleitgase COS/H₂S und das CO₂ jeweils in separaten, nacheinander ablaufenden Absorptionsschritten aus dem Rohsynthesegas abgetrennt. Diese selektive Absorption wird durch geeignete Einstellung der Verfahrensparameter, insbesondere des Mengenverhältnisses von Absorptionsmittel und zu absorbierendem Gas, ermöglicht. Der Vorteil der selektiven Absorption liegt darin, dass das COS/H₂S- und das CO₂-Gas schon bei der Absorption zum größten Teil getrennt gehalten werden und nur der kleinere Teil bei der Regeneration des Methanols getrennt werden muss.

Neben den erwähnten Komponenten COS, H₂S und CO₂ enthält das Rohsynthesegas aber noch weitere Begleitgase. Die Offenlegungsschrift DE 10 2006 056 117 A1 beschreibt ein entsprechendes Verfahren, bei dem den Absorptionsschritten zur Abtrennung der Schwefelkomponenten und des CO₂ ein weiterer Absorptionsschritt vorgelagert ist, um selektiv Metallcarbonyle aus dem Synthesegas abzutrennen.

Enthält der Synthesegasstrom eine zu große Menge an Merkaptanen (C1-C4) und andere unerwünschte, weniger flüchtige, aber im Absorptionsmittel Methanol sehr gut lösliche Schwefelkomponenten, wie z.B. Thioether und/oder Thiophene, können sich diese im Methanol anreichern und werden aufgrund ihres niedrigen Dampfdrucks und ihrer hohe Löslichkeit im Methanol nicht ausreichend aus dem Methanol im Heißregenerator entfernt. Ein zu großer Gehalt dieser Spurenstoffe macht das Methanol für den Waschvorgang unbrauchbar, da es zur Verunreinigung des gewaschenen Synthesegasstromes führen kann. Eine bekannte Möglichkeit, die Anreicherung der unerwünschten Komponenten im Absorptionsmittel zu vermeiden, besteht darin, einen entsprechend großen Teil des Absorptionsmittels kontinuierlich auszutauschen, wobei der dabei ausgetragene Teil entsorgt wird.

### Beschreibung der Erfindung

Aufgabe der Erfindung ist es, ein Verfahren zur Verfügung zu stellen, das die oben beschriebenen Nachteile des Standes der Technik vermeidet. Die Aufgabe wird durch ein Verfahren gemäß Anspruch 1 und durch eine Anlage gemäß Anspruch 7 gelöst.

### Erfindungsgemäßes Verfahren:

Verfahren zur Abtrennung von Begleitgasen aus einem Rohsynthesegasstrom durch Waschen des Rohsynthesegasstroms mit einem flüssigen, physikalisch wirkenden Absorptionsmittel, wobei das Absorptionsmittel in einem Kreisprozess gefahren wird, in welchem es beim Waschen mit Begleitgasen beladen, anschließend durch Druckemiedrigung, Flashen und/oder Erwärmen desorbiert und dann zum Waschen des Rohsynthesegasstroms wiederverwendet wird, dadurch gekennzeichnet, dass als weitere Maßnahme zusätzlich zur Desorption mindestens ein Teil des Absorptionsmittels durch eine ein Adsorbens enthaltende Festkörperschüttung geleitet wird.

### Erfindungsgemäße Anlage:

Anlage zum Abtrennen von Begleitgasen aus einem Rohsynthesegasstrom, umfassend:
- eine Kolonne zur Wäsche des Rohsynthesegasstroms mit einem flüssigen, physikalisch wirkenden Absorptionsmittel,
- einen Behälter zum Austreiben von hauptsächlich Kohlendioxid aus dem beladenen Methanol durch Flashen,
- eine Kolonne zum Austreiben von hauptsächlich Schwefelwasserstoff aus dem beladenen Methanol durch Erwärmen (Heissregenerator), umfassend einen Wärmeaustauscher zur Erwärmung des Absorptionsmittels,
- mindestens einen Adsorptionsbehälter, enthaltend eine Festkörperschüttung mit einem festen Adsorbens,
- wobei der Heissregenerator und der Adsorptionsbehälter in Fluidverbindung stehen.

Unter Fluidverbindung zwischen zwei Bereichen des erfindungsgemäßen Reaktors wird dabei jegliche Art von Verbindung verstanden, die es ermöglicht, dass ein Fluid, beispielsweise der Einsatzgasstrom oder der Synthesegasproduktstrom, von dem einen zu dem anderen der beiden Bereiche strömen kann, unbeachtlich etwaiger zwischengeschalteter Bereiche oder Bauteile.

Die Erfindung ermöglicht eine Verlängerung der Standzeit des Absorptionsmittels. Dadurch werden Kosten für frisches und für die Entsorgung des verunreinigten Absorptionsmittels eingespart.

Die Größe des Anteils des Absorptionsmittels, der durch eine ein Adsorbens enthaltende Festkörperschüttung geleitet wird, wird so eingestellt, dass der Gehalt an unerwünschten Komponenten so niedrig gehalten wird, dass eine Anreicherung der unerwünschten Schwefelkomponenten vermieden wird und das Absorptionsmittel seine Waschfunktion erfüllen kann.

Vorteilhaft ist es, die Adsorptionsanlage mit zwei parallelen Strängen auszuführen, von denen abwechselnd einer in Betrieb ist, während der andere regeneriert wird.

Das Verfahren und eine danach arbeitende Anlage sind besonders geeignet zur Verwendung für die Wäsche von Rohsynthesegas das durch Vergasung von Kohle oder Erdöl erzeugt wurde und als Begleitgase H₂S, COS, HCN, NH₃ und Thiole, wie Merkaptane, Thioether und/oder Thiophene umfasst. Das Waschen von Synthesegas mit leicht flüchtigen Begleitgasen mit einem flüssigen, physikalisch wirkenden Absorptionsmittel ist seit langem bewährt, da das Absorptionsmittel leicht durch Druckabsenkung, Flashen und/oder Erwärmen regenerierbar ist. Durch die erfindungsgemäße, zusätzliche Behandlung des Absorptionsmittels mit einem festen Adsorbens, wird die Verwendbarkeit des Verfahrens auch auf Synthesegase ausgedehnt, die größere Anteile an schwer flüchtigen schwefelhaltigen Begleitgasen haben.

Wenn die Adsorptionskapazität des Aktivkohlebetts erschöpft ist, wird das beladene Bett außer Betrieb genommen und die beladene Aktivkohle durch frische Kohle ersetzt. Alternativ dazu kann das beladene Bett mit Methanoldampf, der beim Erwärmen des Methanols erzeugt wird, regeneriert werden. Der verunreinigt Methanoldampf kann dem aus dem Methanol ausgetriebenen Begleitgasstrom an geeigneter Stelle zugegeben werden.

### Bevorzugte Ausgestaltungen der Erfindung

Bevorzugt erfolgt das Desorbieren durch Erwärmen in einer speziell ausgestalteten Kolonne, einem sog. einem Heissregenerator, und die das Adsorbens enthaltende Festkörperschüttung ist stromabwärts des Heissregenerators angeordnet und steht in Fluidverbindung mit diesem. Hierdurch können gezielt Komponenten, die im Heissregenerator nicht oder nur unzureichend entfernt werden konnten, durch Bindung an das Adsorbens aus dem regenerierten Absorptionsmittel entfernt werden.

Besonders bevorzugt wird das aus dem Heissregenerator ausgeleitete Absorptionsmittel vor dem Zuführen zu der das Adsorbens enthaltenden Festkörperschüttung im indirekten Wärmetausch gegen das dem Heissregenerator zugeführte, beladene Absorptionsmittel gekühlt. Durch die Temperaturabsenkung ist eine vollständigere Entfernung von Störkomponenten mittels Adsorption möglich. Die abgegebene Wärme kann im Sinne einer Wärmeintegration auf das in den Heissregenerator eintretende, noch beladene Absorptionsmittel übertragen werden, wodurch hier Heizenergie eingespart wird.

Eine bevorzugte Ausgestaltung der Erfindung ist dadurch gekennzeichnet, dass als Absorptionsmittel tief gekühltes Methanol verwendet wird. Diese Verfahrensweise ist als Rectisol-Verfahren bekannt und bewährt. Dabei wird die hohe Absorptionsfähigkeit des Methanols bei tiefen Temperaturen ausgenutzt. Daher muss die Anlage mit einer Vorrichtung zum Kühlen des Methanols und mit entsprechender Isolierung ausgestattet sein.

Weitere bevorzugte Ausgestaltungen der Erfindung sind dadurch gekennzeichnet, dass als Adsorbens Aktivkohle oder ein Molekularsieb, d.h. ein Granulat aus künstlich hergestellten Zeolithen, verwendet wird. Welches Adsorbens verwendet wird, hängt von der örtlichen Verfügbarkeit und Marktpreisen und von der Eignung für das jeweils zu reinigende Synthesegas und dessen Begleitgasen ab.

Eine weitere bevorzugte Ausgestaltung der Erfindung besteht darin, dass die Anlage einen Wärmetauscher umfasst, der mit dem Heissregenerator und dem Adsorptionsbehälter in Fluidverbindung steht. Damit wird ein Wärmeaustausch zwischen dem vom Flashbehälter in den Heissgenerator überführten und dem vom Heissgenerator in die Waschkolonne überführten Absorptionsmittels durchgeführt.

Eine weitere bevorzugte Ausgestaltung der Erfindung besteht darin, dass es sich bei dem Absorptionsmittel um tief gekühltes Methanol handelt und die Anlage mit einem Kühlaggregat zur Kühlung des Methanols ausgestattet ist und die tief gekühltes Methanot enthaltenden Apparate mit Isoliermaterial zur Verringerung von Kälteverlusten geschützt sind.

Eine weitere bevorzugte Ausgestaltung der Erfindung besteht darin, dass es sich bei dem festen Adsorbens um Aktivkohle oder ein Molekularsieb handelt. Welches

Adsorbens verwendet wird, hängt von der örtlichen Verfügbarkeit und Marktpreisen und von der Eignung für das jeweils zu reinigende Synthesegas und dessen Begleitgasen ab.

### Ausführungsbeispiel

Weitere Merkmale, Vorteile und Anwendungsmöglichkeiten der Erfindung ergeben sich auch aus der nachfolgenden Beschreibung eines Ausführungsbeispiels und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

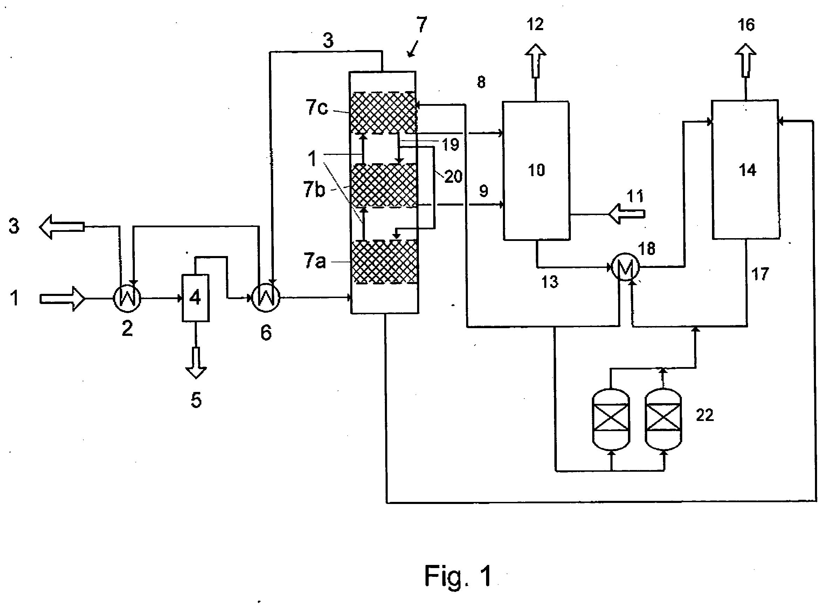

Es zeigt die einzige Figur
- Fig. 1: ein Fließschema einer beispielhaften Ausgestaltung der erfindungsgemäßen Anlage

Anhand von Fig. 1 soll im Folgenden eine beispielhafte Ausgestaltung der Erfindung gemäßen Anlage erläutert werden.

Rohsynthesegas 1 wird in Wärmetauscher 2 gegen das behandelte Synthesegas 3 abgekühlt. In Kondensatabscheider 4 wird das dabei entstandene Kondensat 5 abgeschieden. Nachdem es in Wärmetauscher 6 weiter abgekühlt wurde, wird das Rohsynthesegas in die Waschkolonne 7 eingeleitet. Die Kolonne ist in drei, jeweils ein, Rieselbetten umfassende Waschstufen unterteilt. In der ersten Stufe 7a erfolgt eine Vorwäsche, in der zweiten 7b wird hauptsächlich H2S und in der dritten Stufe 7c wird hauptsächlich CO₂ ausgewaschen. Das so behandelte Synthesegas 3 verlässt danach die Waschkolonne 7, wird in den Wärmetauschern 2 und 6 zur Kühlung des Rohsynthesegases 1 verwendet und dann der weiteren Behandlung aus der Anlage ausgeleitet. Das nur leicht beladene Methanol 8, 9 wird in den Flashbehälter 10 gegeben. Dort wird durch Druckabsenkung und durch Spülen mit Stickstoff 11 das Kohlendioxid 12 aus dem Methanol ausgetrieben und zur weiteren Behandlung aus der Anlage ausgeleitet. Das so vorgereinigte Methanol 13 wird dann in die Kolonne 14 geleitet, in der, durch Erwärmen des Methanols, hauptsächlich schwefelhaltige Begleitgase 14 ausgetrieben werden.

Ebenso wird das Methanol 15, das die dritte und die erste Waschstufe der Waschkolonne 7 durchlaufen hat, in der Kolonne 14 behandelt. Die aus dem Methanol ausgetriebenen, schwefelhaltigen Begleitgase 16 werden aus der Anlage zur weiteren Behandlung, z. B. in einer Claus-Anlage ausgeleitet. Das in Kolonne 14 desorbierte Methanol 17 wird zur Wiederverwendung, über Wärmetauscher 18, in die Waschkolonne 7 zurückgeleitet. Die Ströme 19 und 20 stellen das von oben nach unten durch die Waschstufen laufende Methanol dar. In Wärmetauscher 18 wird ein Wärmeaustausch zwischen dem im Flashbehälter 10 vorbehandeltem Methanol 13 und dem weiter regenerierten Methanol 17 durchgeführt. In dieser Ausgestaltung der Erfindung wird stromwärts nach dem Wärmetauscher 18 ein Teilstrom 21 dem Methanol 17 entnommen und in der Adsorptionsanlage 22 behandelt, indem das Methanol durch ein Aktivkohlebett geleitet wird. Die Adsorptionsanlage 22 besteht in diesem Beispiel aus zwei parallelen Strängen, sodass, auch bei Austausch oder Regenerierung der Aktivkohle in einem der Stränge, der Betrieb der Adsorptionsanlage 22 weiter laufen kann. Der Teilstrom 21 wird anschließend in den Methanolstrom 17 zurück gespeist.

### Gewerbliche Anwendbarkeit

Die Erfindung stellt ein vorteilhaftes Verfahren zur Verfügung, das die Standzeit des Absorptionsmittels und damit die Wirtschaftlichkeit des Absorptionsverfahrens erhöht. Die Erfindung ist daher vorteilhaft gewerblich anwendbar.

### Bezugszeichenliste

- 1: Rohsynthesegas
- 2: Wärmetauscher
- 3: Synthesegas, behandelt
- 4: Kondensatabscheider
- 5: Kondensat
- 6: Wärmetauscher
- 7: Waschkolonne, a,b,c Waschstufen
- 8: Methanol, beladen
- 9: Methanol, beladen
- 10: Flashbehälter
- 11: Stickstoff
- 12: Kohlendioxid
- 13: Methanol, vorgereinigt
- 14: Kolonne (Heissregenerator)
- 15: Methanol
- 16: Begleitgase
- 17: Methanol
- 18: Wärmetauscher
- 19: Methanol
- 20: Methanol
- 21: Methanol
- 22: Adsorptionsanlage

## Patentansprüche

1. Verfahren zur Abtrennung von Begleitgasen aus einem Rohsynthesegasstrom durch Waschen des Rohsynthesegasstroms mit einem flüssigen, physikalisch wirkenden Absorptionsmittel, wobei das Absorptionsmittel in einem Kreisprozess gefahren wird, in welchem es beim Waschen mit Begleitgasen beladen, anschließend durch Druckerniedrigung, Flashen und/oder Erwärmen desorbiert und dann zum Waschen des Rohsynthesegasstroms wiederverwendet wird, **dadurch gekennzeichnet, dass** als weitere Maßnahme zusätzlich zur Desorption mindestens ein Teil des Absorptionsmittels durch eine ein Adsorbens enthaltende Festkörperschüttung geleitet wird.

2. Verfahren gemäß Anspruch 1, **dadurch gekennzeichnet, dass** das Desorbieren durch Erwärmen in einem Heissregenerator erfolgt und die das Adsorbens enthaltende Festkörperschüttung stromabwärts des Heissregenerators angeordnet ist und in Fluidverbindung mit diesem steht.

3. Verfahren gemäß Anspruch 2, **dadurch gekennzeichnet, dass** das aus dem Heissregenerator ausgeleitete Absorptionsmittel vor dem Zuführen zu der das Adsorbens enthaltenden Festkörperschüttung im indirekten Wärmetausch gegen das dem Heissregenerator zugeführte, beladene Absorptionsmittel gekühlt wird.

4. Verfahren gemäß eines der vorigen Ansprüche, **dadurch gekennzeichnet, dass** als Absorptionsmittel tief gekühltes Methanol verwendet wird.

5. Verfahren gemäß eines der vorigen Ansprüche, **dadurch gekennzeichnet, dass** als Adsorbens Aktivkohle oder ein Molekularsiab verwendet wird.

6. Verwendung des Verfahrens gemäß einem der vorherigen Ansprüche zum Waschen von Rohsynthesegas das durch Vergasung von Kohle oder Erdöl erzeugt wurde und als Begleitgase H₂S, COS, HCN, NH₃ und Thiole, wie Merkaptane, Thioether und/oder Thiophene umfasst.

7. Anlage zum Abtrennen von Begleitgasen aus einem Rohsynthesegasstrom, umfassend:
- eine Kolonne zur Wäsche des Rohsynthesegasstroms mit einem flüssigen, physikalisch wirkenden Absorptionsmittel,
- einen Behälter zum Austreiben von hauptsächlich Kohlendioxid aus dem beladenen Methanol durch Flashen,
- eine Kolonne zum Austreiben von hauptsächlich Schwefelwasserstoff aus dem beladenen Methanol durch Erwärmen (Heissregenerator), umfassend einen Wärmeaustauscher zur Erwärmung des Absorptionsmittels,
- mindestens einen Adsorptionsbehälter, enthaltend eine Festkörperschüttung mit einem festen Adsorbens,
- wobei der Heissregenerator und der Adsorptionsbehälter in Fluidverbindung stehen.

8. Anlage gemäß Anspruch 7, ferner umfassend einen Wärmetauscher, der mit dem Heissregenerator und dem Adsorptionsbehälter in Fluidverbindung steht.

9. Anlage gemäß eines der vorigen Ansprüche, **dadurch gekennzeichnet, dass** es sich bei dem Absorptionsmittel um tief gekühltes Methanol handelt und die Anlage mit einem Kühlaggregat zur Kühlung des Methanols ausgestattet ist und die tief gekühltes Methanol enthaltenden Apparate mit Isoliermaterial zur Verringerung von Kälteverlusten geschützt sind.

10. Anlage gemäß eines der vorigen Ansprüche, **dadurch gekennzeichnet, dass** es sich bei dem festen Adsorbens um Aktivkohle oder ein Molekularsieb handelt.
